# EUROPEAN PATENT SPECIFICATION

(11) **EP 2 641 538 B1**
(45) Date of publication and mention of the grant of the patent: **23.03.2016**
(21) Application number: 11841736.9
(22) Date of filing: 11.11.2011
(51) Int. Cl.: A61B 5/107, A61B 5/00, H04N 13/02

(54) **BLOOD VESSEL DISPLAY DEVICE**
BLUTGEFÄSS-ANZEIGEVORRICHTUNG
DISPOSITIF D'AFFICHAGE DE VAISSEAUX SANGUINS

(30) Priority: 15.11.2010 JP 2010254786
(43) Date of publication of application: 25.09.2013
(73) Proprietor: Onishi, Tatsuki, Tokyo 165-0025 (JP)
(72) Inventor: Onishi, Tatsuki, Tokyo 165-0025 (JP)
(74) Representative: Klunker . Schmitt-Nilson . Hirsch
(86) International application number: PCT/JP2011/076019
(87) International publication number: WO 2012/067022

(56) References cited:
- WO-A1-2009/154081
- WO-A2-2008/023155
- WO-A2-2008/136644
- JP-A- 2 172 473
- JP-A- H11 509 748
- JP-A- 2004 267 534
- JP-A- 2006 130 201
- JP-A- 2010 000 218
- US-B1- 6 178 340
- HIROFUMI KANDANI ; TOSHIYUKI UENOYA ; YASUTOMO UETSUJI ; EIJI NAKAMACHI: "Development of blood vessel searching system for HMS", SPIE, PO BOX 10 BELLINGHAM WA 98227-0010 USA, vol. 7055, 27 August 2008 (2008-08-27), XP040441757, DOI: 10.1117/12.793575
- D. GENE LUTHER: 'A head mounted infrared imager for treating the wounded on the battlefield' PROCEEDINGS OF THE 19TH ANNUAL INTERNATIONAL CONFERENCE OF THE IEEE, ENGINEERING IN MEDICINE AND BIO 30 October 1997, pages 722 - 724
- TAKASHI OGAWA: 'Study of Development Design on Vein Injection Support Equipment that uses Prototyping in Design Methods' JOURNAL OF JAPAN SOCIETY OF COMPUTER AIDED SURGERY vol. 10, no. 4, 30 December 2008, pages 521 - 528
- F.P.WIERINGA: 'Remote Non-invasive Stereoscopic Imaging of Blood Vessels: First In-vivo Results of a New Multispe' ANNALS OF BIOMEDICAL ENGINEERING vol. 34, no. 12, December 2006, pages 1870 - 1878

## Description

### Technical Field

The present invention relates to a blood vessel display device that displays a blood vessel existing directly underneath a skin by irradiating the skin with near infrared rays and by photographing the skin irradiated with near infrared rays.

### Background Art

It is known that hemoglobin existing in the blood of a human being has a property of absorbing near infrared rays. Now, a device is proposed that facilitates to recognize how a blood vessel extends (hereinafter, called as "run of a blood vessel") by using this property (for example, Patent Literature 1).

### Citation List

### Patent Literature

Patent Literature 1: Japanese Patent Application Laid-Open No. 2010-148853

### Summary of Invention

### Technical Problem

In the Patent Literature 1, there is disclosed a device that displays a blood vessel by irradiating a skin with near infrared rays and by photographing the near infrared rays reflected therefrom by means of cameras fixed at two places. It becomes possible through the use of this device to recognize the run of a blood vessel even in the case in which the run of the blood vessel is difficult to be visually identified due to the skin color.

However, when cameras are fixed as with the device of the Patent Literature 1, there is a problem that photographing cannot be properly performed depending on the arm thickness. In addition, there also is another problem that, in the case in which the position of cameras is set on the basis of assuming a specified area, it not possible to deal with areas other than the area.

Considering the above-mentioned circumstances, the present invention aims to provide a blood vessel display device that is able to display the run of a blood vessel without being limited to an area.

### Solution to Problem

To solve the above-mentioned problems, a blood vessel display device of the present invention is the blood vessel display device for displaying a blood vessel existing directly underneath a skin by performing near infrared ray photographing of a skin surface in a state of irradiating the skin surface with near infrared rays through a near infrared ray irradiation means, and in being provided with:
a photographing means which includes a first photographing portion having an optical system for performing near infrared ray photographing by using the optical system, and a second photographing portion having an optical system for performing near infrared ray photographing by using the optical system; and
an image display means
   which includes a first image display means displaying a first picture, which is a near infrared ray picture photographed by the above-mentioned first photographing portion, and a second image display means displaying a second picture, which is a near infrared ray picture photographed by the above-mentioned second photographing portion, wherein
at least either one of the above-mentioned first photographing portion and the above-mentioned second photographing portion is provided so that a first optical axis, which is the axis of the optical system of the first photographing portion, and a second optical axis, which is the axis of the optical system of the second photographing portion, intersect with each other on the above-mentioned skin surface, at least either one of a relative relationship between the position of the first optical axis and the position of the second optical axis and a relative relationship between the orientation of the first optical axis and the orientation of the second optical axis is possible to be changed.

According to this blood vessel display device, a first photographing means and a second photographing means are enabled to photograph an identical place by adjusting the position and orientation of the first photographing means and the second photographing means to meet the situation of an object.

Here, the above-mentioned blood vessel display device may be one that is provided with:
a first guide beam irradiation means which irradiates directional visible light in the direction in which the above-mentioned first photographing portion photographs; and
a second guide beam irradiation means which irradiates directional visible light in the direction in which the above-mentioned second photographing portion photographs, wherein
by allowing visible light irradiated from the above-mentioned first guide beam irradiation means and visible light irradiated from the above-mentioned second guide beam irradiation means to intersect with each other on a skin surface, the above-mentioned first optical axis and the above-mentioned second optical axis are enabled to intersect with each other on the above-mentioned skin surface.

According to this blood vessel display device, it is possible to adjust so that the first photographing means and the second photographing means photograph an identical place on the basis of beam-like visible light irradiated by the first guide beam irradiation means and the second guide beam irradiation means.

In addition, the blood vessel display device may be a device in which the first photographing portion and the second photographing portion are arranged so that the first optical axis and the second optical axis are included in optical path planes, which are an identical plane, and at least either one of the first photographing portion and the second photographing portion is provided so that at least either one of a relative relationship between the position of the first optical axis and the position of the second optical axis and a relative relationship between the direction of the first optical axis and the direction of the second optical axis is possible to be changed while a state in which the first optical axis and the second optical axis are included in the optical path plane is maintained.

According to this blood vessel display device, since the movement direction and orientation of the above-mentioned first photographing means and the above-mentioned second photographing means are restricted, it is possible to adjust so that the first photographing means and the second photographing means photograph an identical place with a simple operation.

Further, the blood vessel display device may include an adjustment mechanism for changing positions and directions of both the first optical axis and the second optical axis while maintaining the relative relationship between the position of the first optical axis and the position of the second optical axis and also the relative relationship between the direction of the first optical axis and the direction of the second optical axis.

According to this blood vessel display device, it is possible to properly adjust the position and orientation of the first photographing means and the second photographing means by use of the adjustment mechanism described above.

Moreover, the above-mentioned blood vessel display device may be such one that the above-mentioned image display means is one in which the above-mentioned first image display means and the above-mentioned second image display means are integrally formed, and may also be such one that includes a parallax formation optical member allowing only first light of the first light from the first image display means and second light from the second image display means to head for a predetermined first viewpoint and also allowing only the second light to head for a predetermined second viewpoint.

This blood vessel display device is one that is able to display the above-mentioned first picture and second picture on left and right eyes, respectively. If appropriate parallax information is obtained from these pictures, a user can perceive the position of a blood vessel. That is, the user can adjust the position and orientation of the above-mentioned photographing means so as to be able to perceive the position of the blood vessel on one's own while seeing the above-mentioned image display means. Incidentally, the above-mentioned optical member for forming parallax may be one that changes the courses of the first light and the second light so that only the first light of the above-mentioned first light and the above-mentioned second light heads for the above-mentioned first viewpoint and only the second light heads for the above-mentioned second viewpoint, or may be one that cuts off partial rays of the first light and partial rays of the second light.

Additionally, the blood vessel display device include a position information obtaining means which obtains information of a position of the first optical axis and a position of the second optical axis, a direction information obtaining means which obtains information of a direction of the first optical axis and a direction of the second optical axis, and a depth information display means which displays in a state in which the first optical axis and the second optical axis intersect with each other at an identical place on the skin surface, information for estimating the depth of a blood vessel existing underneath the identical place on either one of the first image display means and the second image display means by using information obtained through the position information obtaining means and the direction information obtaining means.

According to this blood vessel display device, it is possible to display information for estimating a blood vessel depth and to allow a user to easily grasp the run of a blood vessel.

The blood vessel display device may be a device in which the depth information display means displays information for estimating the depth of a blood vessel, which exists at the identical place and also exists on the optical axis of either one of the first optical axis and the second optical axis, on either one of the first image display means and the second image display means by using the information obtained through the position information obtaining means and the direction information obtaining means.

According to this blood vessel display device, it is possible to estimate a blood vessel depth by limiting the conditions required for estimating the blood vessel depth through adjusting the position and orientation of the above-mentioned photographing means so that a blood vessel is arranged along the above-mentioned first optical axis and the above-mentioned second optical axis

The blood vessel display device may include an input means which inputs as to a near infrared ray picture displayed on either one of the first image display means and the second image display means, positional information of a blood vessel existing underneath the identical place in the picture, in which the depth information display means may display information obtained through the position information obtaining means and the direction information obtaining means, and information for estimating the depth of the blood vessel by using the blood vessel positional information inputted by the input means on either one of the first image display means and the second image display means.

According to this blood vessel display device, it is possible to obtain information for estimating the position of a blood vessel by means of information inputted from the above-mentioned input means and to display information for estimating the blood vessel depth.

### Advantageous Effects of Invention

According to a blood vessel display device of the present invention, it is possible to provide a blood vessel display device that is able to display the run of a blood vessel without being restricted by an area.

### Brief Description of Drawings

Fig. 1 is a perspective view showing a blood vessel display device of the present embodiment.
Figs. 2(a) to 2(b) are drawings showing a main photographing unit and a sub-photographing unit.
Fig. 3 is a drawing showing a manner of adjusting the photographing direction of photographing units.
Figs. 4(a) to 4(b) are cross-sectional views showing near infrared rays irradiated on a skin surface and the reflected light thereof.
Figs. 5(a) to 5(b) are drawings showing an example of a skin photographed using near infrared rays.
Figs. 6(a) to 6(c) are drawings showing arteries and veins run underneath the skin of a forearm portion.
Fig. 7 is a drawing showing a manner of photographing a skin surface while changing the viewpoint.
Figs. 8(a) to 8(e) are drawings showing five viewpoints shown in Fig. 7 and the images photographed at the respective viewpoints.
Fig. 9 is a drawing showing the configuration of an image display device in the first variation example of the blood vessel display device 1.
Fig. 10 is a drawing showing the configuration shown in Fig. 9 by separating into the case of the right eye and the case of the left eye.
Figs. 11(a) to 11(b) are drawings showing a pinhole camera model.
Figs. 12 (a) to 12(c) are drawings showing a manner in which a skin surface is photographed by a main photographing unit and a sub-photographing unit.
Figs. 13(a) to 13(b) are drawings showing the positional relationship in Figs. 12(a) to 12(c) by using a camera coordinate system having the main photographing unit at the center thereof.
Fig. 14 is a flowchart showing the program flow for displaying additional information.
Fig. 15 is a drawing showing a manner in which scales are indicated in accordance with the flowchart shown in Fig. 14.
Figs. 16 (a) to 16(c) are drawings showing usage examples of the second variation example of the blood vessel display device.

### Description of Embodiments

An embodiment of a blood vessel display device of the present invention will be described below using the drawings.

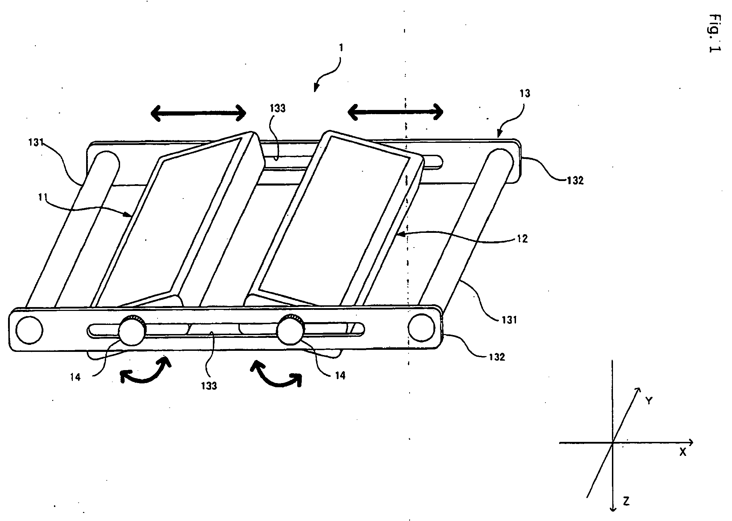

Fig. 1 is a perspective view showing a blood vessel display device of the present embodiment.

The blood vessel display device 1 shown in Fig. 1 is provided with a main photographing unit 11, a sub-photographing unit 12 (corresponding to one example of the photographing means of the present invention) and a rectangular outer frame 13 for supporting these two photographing units. Incidentally, for convenience of explanation, a three-dimensional coordinate system constituted of three axes of X-axis, Y-axis and Z-axis orthogonal to each other is shown at the lower right of Fig. 1, and explanations will be given from here with reference to this coordinate system as appropriate.

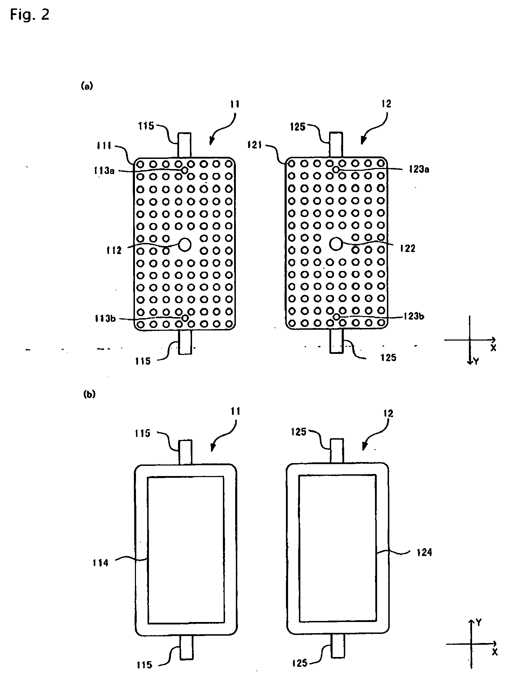

The main photographing unit 11 (corresponding to the first photographing portion of the present invention) has a flat rectangular parallelepiped shape and provided with a digital video camera capable of near infrared ray photographing (hereinafter, simply called as "camera"), a liquid crystal display device for displaying pictures of this camera, and a shaft for mounting this main photographing unit 11 to an outer frame 13 (refer to Figs. 2(a) to 2(b). The camera is integrated in the main photographing unit 11 and the lens of this camera is disposed at the center of one side face of the main photographing unit 11. Likewise, the liquid crystal display device is disposed on the face opposite to this face on which the lens is disposed. Hereinafter, the face on which the lens is disposed is called as the front side, and the face on which the liquid crystal display device is disposed is called as the back side. The shaft is provided on the both ends in the longitudinal direction of the main photographing unit 11.

The sub-photographing unit 12 (corresponding to the second photographing portion of the present invention) is of a similar shape to that of the above-mentioned main photographing unit 11, and is provided with a near infrared ray photographing camera, a liquid crystal display device and shafts. As to these configurations, the explanation is omitted since being similar to the main photographing unit 11. Incidentally, hereinafter, there are cases in which the main photographing unit 11 and the sub-photographing unit 12 are generically called as photographing units. The detail of the photographing units 11, 12 will be described later using Figs. 2(a) to 2(b.

The rectangular outer frame 13 is provided with two guide rails 132, which constitute the sides in the long-hand direction, and two support struts 131, which constitute the sides in the short-hand direction. Incidentally, hereinafter, this explanation will be continued on the understanding that the guide rails 132 are parallel to X-axis and the support struts 131 are parallel to Y-axis.

In each of the two guide rails 132, there is formed a clearance 133 along the long-hand direction. In these clearances 133, the shafts provided on the both ends of the photographing units 11, 12 are inserted, and the photographing units 11, 12 are in a state of being pinched by the two guide rails 132. Here, the photographing units 11, 12 are arranged so that movement along the clearances 133 of the guide rails 132 and rotation having the shafts as the central axis are enabled while the state in which the above-mentioned shafts are parallel to Y-axis is maintained. That is, the photographing units 11, 12 are each arranged so that adjustment of the position by means of movement in the X-axis direction (refer to the arrows in a left-to-right direction in the drawing) between the two guide rails 132 is enabled, and adjustment of the orientation by means of rotation having an axis parallel to Y-axis as the center (refer to the arrows indicated around screws 14 in the drawing) is also enabled.

On the top end of the above-mentioned shafts, there are mounted adjustment screws 14 for fixing the photographing units 11, 12 to the guide rails 132. The position and orientation of the photographing units 11, 12 can be adjusted by loosening these adjustment screws 14, and the position and orientation of the photographing units 11, 12 can be fixed by tightening the adjustment screws 14.

Then, an explanation on the main photographing unit 11 and the sub-photographing unit 12 will be explained using Figs. 2(a) to 2(b). The figures are drawings showing the main photographing unit 11 and the sub-photographing unit 12. Incidentally, in these drawings, a state in which the front sides of the both ones face toward the Z-axis direction is shown.

In Fig. 2(a), the front side of the main photographing unit 11 and the sub-photographing unit 12 is shown.

In the front side of the main photographing unit 11, there are provided near infrared ray light emitting diodes (LEDs) 111 arranged in almost whole of this front side, a lens 112 arranged in the center, and two visible light LEDs 113a, 113b (corresponding to one example of the first guide beam irradiation means of the present invention) arranged in the upper and lower portions. The two visible light LEDs 113a, 113b and the above-mentioned lens 112 are arranged in a straight line in the Y-axis direction.

In the front side of the sub-photographing unit 12, there are provided near infrared ray LEDs 121, a lens 122, and visible light LEDs 123a, 123b (corresponding to one example of the second guide beam irradiation means of the present invention) as with the main photographing unit 11. As to these configurations, the explanation is omitted since being similar to the main photographing unit 11.

In Fig. 2(b), the back side of the main photographing unit 11 and the sub-photographing unit 12 is shown. In the back side of the main photographing unit 11, there is provided a liquid crystal display device 114 (corresponding to one example of the first image display means of the present invention). Likewise, also in the back side of the sub-photographing unit 12, there is provided a liquid crystal display device 124 (corresponding to one example of the second image display means of the present invention).

In Figs. 2(a) and (b), there are shown shafts 115, 125 protruding in the long-hand direction of each of the main photographing unit 11 and the sub-photographing unit 12. These shafts 115, 125 are inserted in the clearances 133 of the guide rails 132, and maintain a state of being parallel to the Y-axis. Incidentally, the orientation of the front side of each of the photographing units 11, 12 is rendered to be an orientation orthogonal to the central axis of each of the shafts 115, 125.

The near infrared ray LEDs 111, 121 are ones for irradiating a skin with near infrared rays required for near infrared ray photographing. Incidentally, these near infrared ray LEDs 111, 121 are not limited to ones for applying only near infrared rays, but may be ones that simultaneously apply, for example, light having a wavelength other than that of the near infrared ray.

The lenses 112, 122 are camera lenses provided in the inside of the photographing units 11, 12, respectively. The camera is a small-size one used for a cellular phone or the like, and includes a light reception element that responds to light having wavelengths from visible light to near infrared rays. To the lenses 112, 122, a near infrared ray transmission filter is attached to remove light having a wavelength other than that of near infrared rays. That is, the photographing units 11, 12 are ones that are made capable of near infrared ray photographing by allowing an ordinary small-size camera to light-receive only near infrared rays. Near infrared ray pictures photographed by these cameras are displayed on the liquid crystal display devices 114, 124 provided on the back sides, respectively, as shown in Fig. 2(b). Incidentally, the optical axes of the lenses 112, 122 are arranged to be vertical with respect to the front sides of the photographing units 11, 12, respectively.

Here, consideration is taken on the optical axes of the photographing units 11, 12 including the configuration explained above. These optical axes are axes that pass through the centers of the lenses 112, 122 located on the front sides of the photographing units 11, 12 and intersect with respect to the central axes of the shafts 115, 125, respectively.

In the case when the photographing units 11, 12 have moved toward the X-axis direction without changing the orientation, the above-mentioned optical axes move toward the X-axis direction, i.e., the direction orthogonal with respect to the shafts 115, 125 at the time before the movement. Likewise, in the case when the photographing units 11, 12 have rotated without changing the position, having the shafts 115, 125 as the central axes, the above-mentioned optical axes are to change the directions in the planes orthogonal to the extending directions of the shafts 115, 125, respectively. That is, the above-mentioned optical axes are to move in the planes orthogonal with respect to the shafts 115, 125 due to the movement and rotation of the photographing units 11, 12, respectively. Hereinafter, this plane is called as an optical path plane.

That is, although the photographing units 11, 12 are enabled to move in the X-axis direction and to rotate having the shafts 115, 125 parallel to the Y-axis as the central axes, respectively, the above-mentioned optical path plane does not change due to these movement and rotation.

The main photographing unit 11 and the sub-photographing unit 12 provided in the blood vessel display device 1 of the present embodiment are different from each other only in their positions in the X-axis direction. That is, the positional relationship between the main photographing unit 11 and the sub-photographing unit 12 can be considered similarly to the case of having moved in the X-axis direction, explained in the above description. Accordingly, the optical path planes of the respective main photographing unit 11 and sub-photographing unit 12 are an identical plane, and the both optical axes are to be included in the identical plane. The both optical axes are thereby arranged to be easily adjusted so that the main photographing unit 11 and the sub-photographing unit 12 are able to photograph an identical area.

In the case of adjusting the both optical axes of the main photographing unit 11 and the sub-photographing unit 12, it is thought that a user carries out the adjustment while seeing the liquid crystal display devices 114, 124 respectively provided thereon since not possible to see the optical axes. However, the operation of adjusting the optical axes while comparing two images with eyes takes time and causes a problem of becoming a burden of the patient. The above-mentioned visible light LEDs 113, 123 are ones provided for the reason of facilitating adjustment of the optical axes of the photographing units 11, 12 and resolving this problem.

The visible light LEDs 113, 123 are ones that apply a laser beam from the front side of the respective photographing units 11, 12 in the direction vertical thereto. These visible light LEDs 113, 123 are arranged individually in the upper and lower portions of the front sides of the photographing units 11, 12, as shown in Fig. 2(a). The visible light LEDs 113, 123 and the lenses 112, 122 are arranged so as to be in a straight line in the Y-axis direction on the front sides of the photographing units 11, 12.

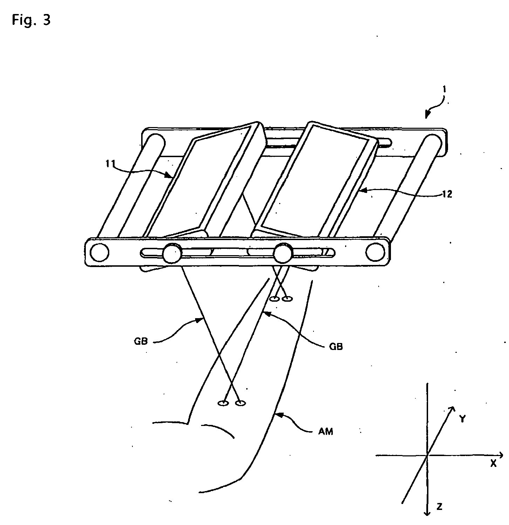

The visible light LED 113a provided on one end side in the extending direction of the shaft 115 on the front side of the main photographing unit 11 (the upper edge side in Figs. 2(a) to 2(b)) is paired with the visible light LED 123a provided on one end side in the extending direction of the shaft 125 on the front side of the sub-photographing unit 12 (the upper edge side in Figs. 2(a) to 2(b)), and is arranged so that the laser beams irradiated from them intersect with each other. Likewise, the visible light LED 113b provided on another end of the main photographing unit 11 (the lower edge side in Figs. 2(a) to 2(b)) opposite to the end in which the above-mentioned visible light LED 113a is provided is paired with the visible light LED 123b provided on another end of the sub-photographing unit 12 (the lower edge side in Figs. 2(a) to 2(b)) opposite to the end in which the above-mentioned visible light LED 123a is provided, and is arranged so that the laser beams irradiated from them intersect with each other. When these laser beams are brought to intersect with each other, the mid-point of the straight line linking the intersection points becomes the intersection point of the respective optical axes of the main photographing unit 11 and the sub-photographing unit 12. Accordingly, it is possible to estimate the intersection point of the above-mentioned optical axes on the basis of the intersection point of these laser beams, and it becomes possible to adjust the invisible optical axis to a certain extent without seeing the liquid crystal display devices 114, 124. As to this one example, an explanation will be given by using Fig. 3.

Fig. 3 is a drawing showing a manner of adjusting the photographing directions of the photographing units 11, 12. Incidentally, the explanation below is given with the assumption that a skin surface is a flat plane parallel to the XY plane.

In Fig. 3, there are presented a forearm portion AM and a blood vessel display device 1 of the present embodiment used with respect to this forearm portion AM. Moreover, there is presented a manner in which laser beams GB are irradiated from the visible light LEDs 113, 123 provided in the respective photographing units 11, 12 to both the wrist side and the elbow side. The two laser beams GB irradiated to the wrist side intersect with each other immediately above the skin. Likewise, the two laser beams GB irradiated to the elbow side also intersect with each other immediately above the skin. As explained in the above description, from the fact that the mid-point of the straight line linking the intersection points of the respective laser beams GB becomes the intersection point of optical axes, it is known that the intersection point of the respective optical axes of the main photographing unit 11 and the sub-photographing unit 12 exists immediately above the skin. In the case of trying to photograph a parallax image on the basis of a skin surface, the position and orientation of each of the photographing units 11, 12 may be adjusted so that the intersection points of these laser beams GB intersect at the skin surface.

In the explanation in the above description, the explanation has been given with the assumption that the skin surface is one parallel to the XY plane. Although the skin surface is actually not a flat plane, since the blood vessel display device 1 of the present embodiment is not one for accurately measuring the position of a blood vessel, it is enough to be able to adjust optical axes as aimed to some extent. Moreover, in the case in which the skin is inclined with respect to the XY plane as with the case, for example, in which the wrist is lifted in Fig. 3, the two intersection points of laser beams do not intersect at the skin surface. In this case, the position of the forearm portion AM may be adjusted or the position and orientation of the whole of the blood vessel display device 1 may be adjusted so that the two intersection points will intersect with each other at the skin surface.

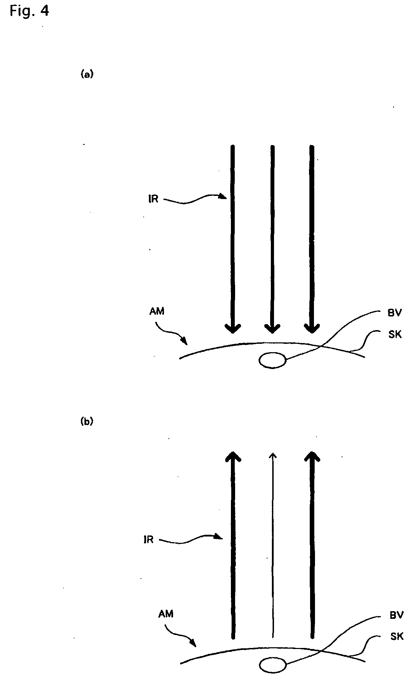

When the position and orientation of the photographing units 11, 12 are adjusted through the operation having been explained by using Fig. 3, in succession thereto, near infrared rays are irradiated to the skin surface by means of the near infrared ray LEDs 111, 121, and near infrared ray photographing is carried out. As to this principle, an explanation will be given by using Figs. 4(a) to (b). The figures are cross-sectional views showing near infrared rays irradiated on a skin surface and the reflected light thereof. It is noted that the difference in strengths of near infrared rays is indicated by the difference in thicknesses of lines.

The manner that near infrared rays IR are irradiated with respect to the forearm portion AM is shown in Fig. 4(a), and the reflected light thereof is shown in Fig. 4(b). When near infrared rays IR impinge on a skin SK, a portion thereof is reflected. Moreover, while the near infrared rays IR transmit into the skin SK, a portion thereof is to be reflected. Here, when the near infrared rays IR impinge on a blood vessel BV, the near infrared rays IR is not to be reflected, since the near infrared rays IR are absorbed by hemoglobin in the blood. That is, as compared with the case in which the near infrared rays IR do not impinge on the blood vessel BV, the reflected near infrared rays IR become weaker. In Fig. 4(b), a manner is shown that the reflected light of the near infrared rays IR irradiated with respect to a blood vessel BV has become weaker as compared with the reflected light of the portion irradiated to the other portion. It becomes possible to display the blood vessel BV by performing near infrared ray photographing of this reflected light.

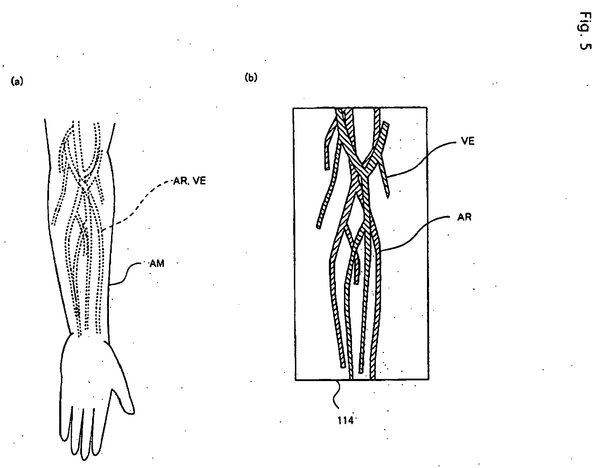

Figs. 5(a) to 5(b) are drawings each showing an example of a skin photographed using near infrared rays.

A forearm portion AM is shown in Fig. 5(a), and arteries AR and veins VE existing under the skin are shown by dotted lines. In addition, the forearm portion AM photographed using near infrared rays IR is shown in Fig. 5(b), and blood vessels, which are darkly displayed in actuality, are indicated by hatching in the drawing. As explained in the above description, since the portion of blood vessels is darkly displayed, run of a blood vessel can thereby be grasped.

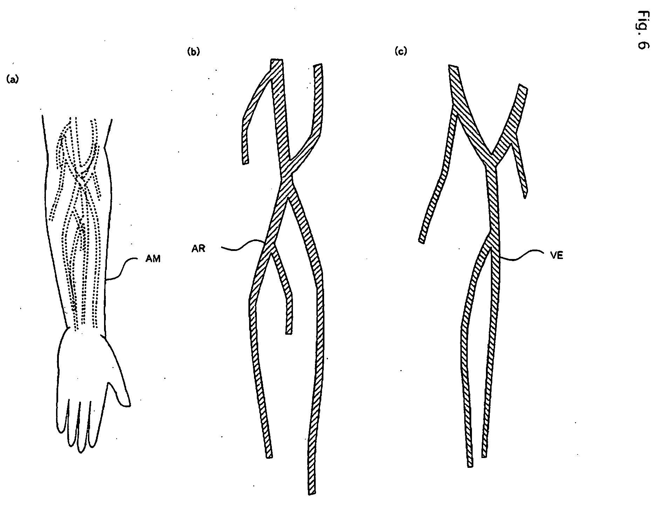

Here, an explanation will be given by using Figs. 6(a) to 6(c) to Figs. 8(a) to 8(e) about how photographed images changes depending on the position of a camera (hereinafter, called sometime as "viewpoint") and the orientation thereof.

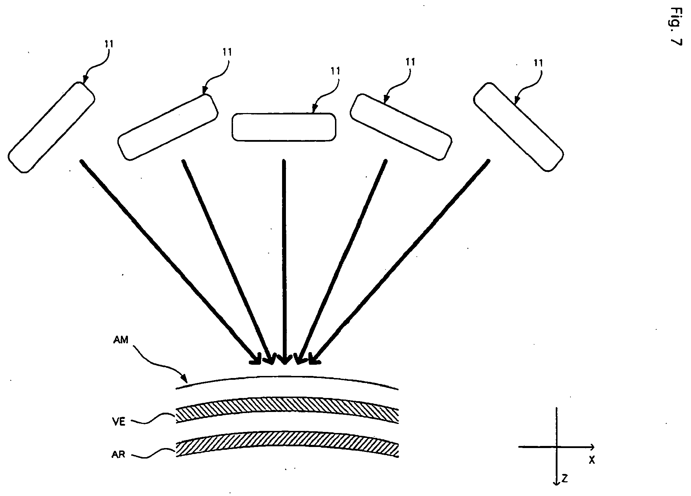

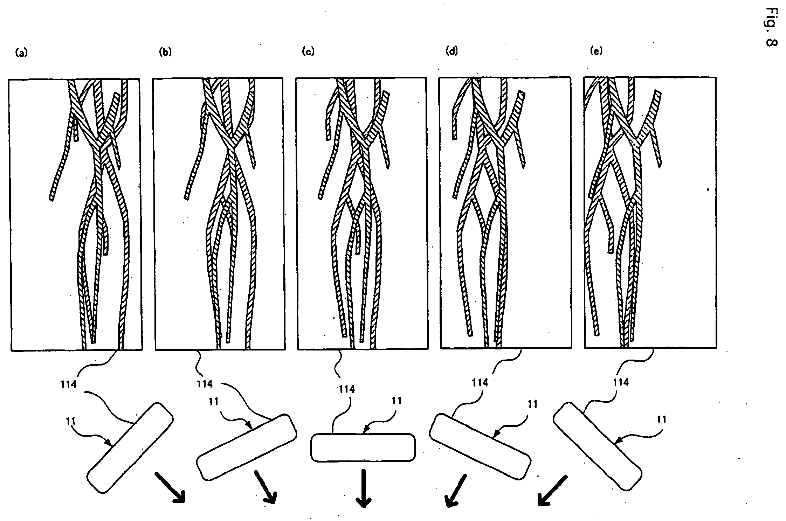

Figs. 6(a) to 6(c) are drawings showing arteries AR and veins VE run underneath the skin of a forearm portion AM. In Fig. 6(a), the forearm portion AM and blood vessels running underneath the skin thereof are shown as with Fig. 5(a). In addition, the arteries AR shown in Fig. 6(a) is indicated by hatching declined to the left in Fig. 6(b), and the veins VE shown in Fig. 6(a) is indicated by hatching declined to the right in Fig. 6(c). The veins VE run in a portion closer to the skin as compared with the arteries AR. Here, as to the case of moving the main photographing unit 11 so as to cross with respect to the forearm portion AM shown in Fig. 6(a), an explanation will be given by using Fig. 7 and Figs. 8(a) to 8(e).

Fig. 7 is a drawing showing a manner of photographing a skin surface while changing the viewpoint.

In Fig. 7, there is shown a manner that the forearm portion AM is photographed from five viewpoints. Here, the main photographing unit 11 is assumed so that the position and orientation thereof are adjusted to photograph the identical position. In Fig. 7, it is indicated by hatching declined to the right that a layer where the veins VE run exists at a location close to the skin. In addition, it is indicated by hatching declined to the left that a layer where the arteries AR run exists at a location far from the skin.

Figs. 8(a) to 8(e) are drawings showing five viewpoints shown in Fig. 7 and the images photographed at the respective viewpoints.

In Figs. 8(a) to 8(e), images photographed on the bases of five viewpoints shown in Fig. 7 are shown. Incidentally, in order to make the corresponding relationship between the viewpoints and the images understandable, the main photographing unit 11 shown in Fig. 7 is shown again. When seeing a group of these images in order from left to right, the veins VE existing in a location near the skin surface have not moved much from the center of the screen, but the arteries AR existing in a location far from the skin surface have moved much. The difference in movement is due to the difference in distances to the main photographing unit 11. That is, in a group of photographed images when photographing an identical location as with this example, a blood vessel existing directly underneath a skin moves a bit and the other blood vessels are to move to a large extent with distance from the skin. It is possible to grasp the blood vessel depth by the difference in these movements to a certain extent.

Although an example of using the main photographing unit 11 is taken up in the explanation described above, it is possible to obtain identical images by using not only the main photographing unit 11 but also the sub-photographing unit 12. Accordingly, if both the main photographing unit 11 and the sub-photographing unit 12 are adjusted to be appropriately positioned and oriented, it becomes possible to estimate the blood vessel depth as comparing two images.

For example, there is an adjustment method in which the position and orientation of the sub-photographing unit 12 are adjusted after an objective blood vessel has been displayed so as to run the center of the liquid crystal display device 114 in the Y-axis direction by adjusting the main photographing unit 11. When adjusted like this, the blood vessels displayed on the sub-photographing unit 12 are positioned at different distances from the central portion depending on the depth, and the depth is therefore easily estimated. That is, it is possible to use the main photographing unit 11 for the sake of grasping the outline of an objective blood vessel, and to use the sub-photographing unit 12 for the sake of grasping the depth of the objective blood vessel.

The usage method of the main photographing unit 11 and the sub-photographing unit 12 explained in the above description is one example, and information of blood vessels may be obtained through the other usage methods. That is, the blood vessel display device 1 is one that photographs plural images (parallax images) from different positions by means of the main photographing unit 11 and the sub-photographing unit 12, and is able to obtain information about the blood vessel depth on the basis of those images.

According to the blood vessel display device 1 explained in the above description, it is possible to carry out an adjustment so that the main photographing unit 11 and the sub-photographing unit 12 photograph an identical portion by rendering the intersection positions of laser beams, which are irradiated from the visible light LEDs 113, 123 provided in the main photographing unit 11 and the sub-photographing unit 12, to be indicators. At this occasion, since the photographing units 11, 12 are each limited so as to be able to move only with respect to a single axis and to rotate also only with respect to a single axis by means of the guide rails 132 and the shafts 115, 125, the adjustment of the position and orientation of each of the photographing units 11, 12 is easy. Incidentally, although images are displayed on the liquid crystal display devices 114, 124 in the blood vessel display device 1 of the present embodiment, these images may be displayed by using external monitors or the like. Likewise, the near infrared ray LEDs 111, 121 may be separated from the photographing units 11, 12.

Here, a first variation example of the blood vessel display device 1 explained in the above description will be explained. The first variation example explained below is one that allows the left and right eyes of a user to perceive respective images and allows the user to be able to easily obtain parallax information by using an image display device for enabling to perceive different images depending on viewpoints. First, an outline of this image display device will be explained using a drawing, and the first variation example of the blood vessel display device 1 will be explained based on this explanation.

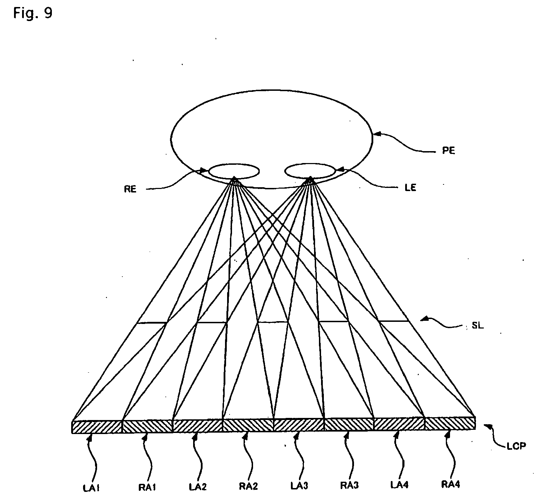

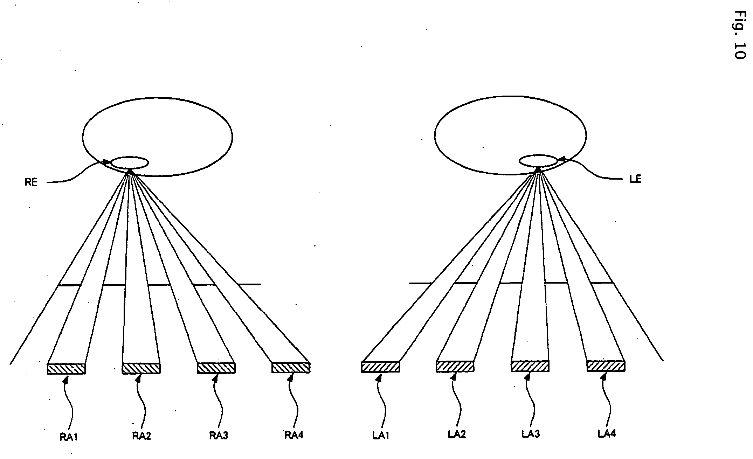

Fig. 9 is a drawing showing the configuration of an image display device in the first variation example of the blood vessel display device 1. Likewise, Fig. 10 is a drawing showing the configuration shown in Fig. 9 by separating into the case of the right eye and the case of the left eye. In Fig. 9, there are shown a liquid crystal panel LCP that displays an image by allowing pixels to light-emit, and slits SL provided on the side of a user who sees this liquid crystal panel LCP. Furthermore, there is also shown the user PE who sees the liquid crystal panel LCP through these slits SL. Incidentally, these slits SL are formed of liquid crystals and are configured so as to adjust the location and size of the area cutting off light, or so that the whole of the liquid crystal panel LCP can be directly seen by not permitting to cut off light.

When the user PE shown in Fig. 9 sees the liquid crystal panel LCP through the slits SL, areas RA1 to RA4 (the portions indicated by hatching declined to the right) are seen from the right eye RE of the user PE, and areas LA1 to LA4 (the portions indicated by hatching declined to the left) are seen from the right eye RE. In Fig. 10(a), it is shown that the areas RA1 to RA4 are seen from the right eye RE of the user PE, and in Fig. 10(b), it is shown that the areas LA1 to LA4 are seen from the left eye LE of the user PE. That is, by means of these slits SL, the liquid crystal panel LCP can be separated into the areas RA1 to RA4 for displaying an image for the right eye and the areas LA1 to LA4 for displaying an image for the left eye. By using these areas, it becomes possible to allow the user to perceive the image for the right eye and the image for the left eye by means of a single image display device. Incidentally, it is possible to separate these areas more appropriately by adjusting the locations of the area cutting off light. In addition, although the image display device explained here is one using a parallax barrier method, as long as being one that enables to allow the user to perceive an image for the right eye and another image for the left eye by means of a single image display device like this, one using, for example, a lenticular method may also be adopted.

Here, an explanation will be given as to the first variation example of the blood vessel display device 1. This first variation example is one in which an image display device including the configuration explained in the above description is adopted as the liquid crystal display device 114 of the main photographing unit 11. Incidentally, in this first variation example, this image display device corresponds to an example of the image display means of the present invention in which a first image display means and a second image display means are integrally formed. Moreover, the slits SL of this image display device correspond to an example of the parallax formation optical member of the present invention. By this liquid crystal display device 114, for example, it becomes possible to allow the right eye to perceive a blood vessel photographed by the camera of the main photographing unit 11 and also to allow the left eye to perceive the blood vessel photographed by the camera of the sub-photographing unit 12. At this time, if images similar to parallax images that a user naturally views are perceived by the right eye and the left eye, respectively, the user can easily grasp the depth of the blood vessel. In addition, it is also possible to adjust the position and orientation of each of the photographing units 11, 12 while seeing the liquid crystal display device 114 of the main photographing unit 11 so that the user can easily grasp the blood vessel depth by oneself. Moreover, although it is necessary to move the viewpoint in order to see both screens in the case of using two liquid crystal display devices, such a movement of the viewpoint is circumvented in the case of the present first variation example. The above description is the explanation as to the first variation example.

It is thought to use a fixed camera in order to accurately know the position of a blood vessel, but there is a fear that the camera cannot be used except for only a specified portion when the camera is fixed. Moreover, in the case in which the optical axis of the camera is rendered in a state nearly parallel with respect to a skin surface, it is thought that blood vessels running at an identical depth are displayed by being overlapped and proper parallax information cannot be obtained. The blood vessel display device 1 of the present embodiment can be used for various portions since the position and orientation of the photographing units 11, 12 can be adjusted. Depending on objects, it is also possible to use the blood vessel display device 1 by fixing to an instillation stand or the like (corresponding to one example of the adjustment mechanism of the present invention), which is able to adjust the position and angle of the whole thereof. Although there is a fear that measurement of the accurate position of a blood vessel is apt to become difficult due to that the photographing units 11, 12 are rendered movable, it is useful enough if the position of a blood vessel and the depth thereof from a skin are roughly known from parallax images in the medical practice of intravenous injection or the like, and it therefore is not necessary to fix the camera all the time. Likewise, although a method of measuring the position of a blood vessel by performing a complicated computation like image processing through use of information processing equipment is also thought, the apparatus becomes a large scale and also costly. Since being of simple configuration and also not costly, the blood vessel display device 1 of the present embodiment is expected to become prevalent in more medical fields.

The blood vessel display device 1 of the present embodiment uses near infrared rays, and therefore does not injure a patient and enables to lessen the load on the patient. In addition, the blood vessel display device 1 is useful in the case in which blood vessels are difficult to be visually recognized as with those of a highly obese person and a patient having a skin discolored due to a disease. Furthermore, the blood vessel display device 1 lessens the opportunity that a beginner who is just involved with medical care fails and also enables to lessen the opportunity of giving a pain to a patient. The blood vessel display device 1 of the present embodiment is allowed to be used while actually sticking an injection needle; at this occasion, it is favorable that the injection needle is constituted of a raw material visually recognizable by near infrared ray photographing. Specifically, it is favorable that a material easily absorbing near infrared rays as compared with a human body (for example, a skin and blood vessels) or, conversely, a material easily reflecting near infrared rays as compared with a human body (for example, a skin and blood vessels) is used for the injection needle. Since near infrared rays are used, it is also possible to use medical equipment using, for example, ultrasonics in combination therewith.

Up to here, an explanation as to the basic configuration of the blood vessel display device 1 of the present invention has been given. In the next place, an explanation as to a second variation example of this blood vessel display device 1 will be given.

The second variation example explained below is such that the photographing units 11, 12 of the blood vessel display device 1 explained in the above description are each provided with a sensor for knowing the position and orientation thereof, and display information for estimating the depth of blood vessels on the basis of the information from these sensors. The object photographed by a camera and the photographed contents relate to parameters such as the positional relationship between the camera and the object, the orientation of the camera, the lens of the camera, and the like. By using the information, it becomes possible to derive the relationship between movement amounts of blood vessels due to parallax and actual depths of blood vessels as shown in Figs. 8(a) to 8(e). The second variation example explained below is one that presents information as to the depth of blood vessels from the information as to the camera and the photographed contents on the basis of a pinhole camera model. Here, an explanation as to the relationship between the depth of blood vessels and parallax images based on the pinhole camera model will be given.

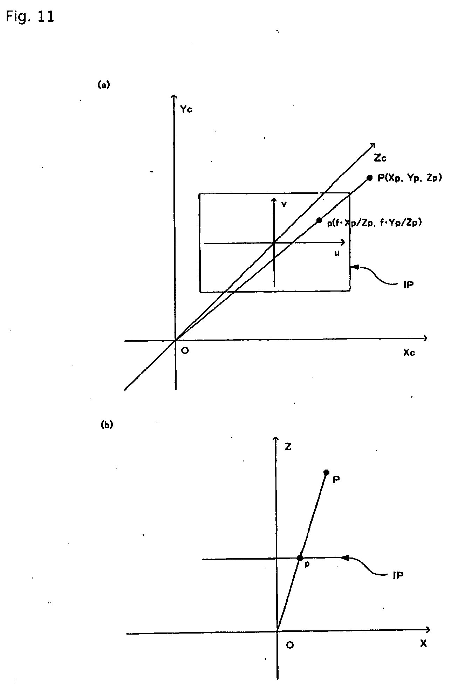

Figs. 11(a) to 11(b) are drawings showing a pinhole camera model.

In Fig. 11(a), there are shown a three-dimensional coordinate system constituted of three axes of Xc-axis, Yc-axis and Zc-axis, and an image plane IP constituted of two axes of u-axis and v-axis. This coordinate system is also called as a camera coordinate system, and is a coordinate system in which the viewpoint of a camera is rendered to be the origin point and the optical axis thereof is rendered to be Zc-axis. A three-dimensional coordinate photographed by this camera is projected at a coordinate on the image plane IP apart from the origin point by a distance f in the Zc-axis direction. In this drawing, it is shown that a three-dimensional point P (Xp, Yp, Zp) is projected at a point p (f·Xp/Zp, f ·Yp/Zp) of the image plane IP. Here, the distance f from the origin point to the image plane IP is an internal parameter of the camera and is defined by the lens performance and the like. That is, the coordinate projected in the image plane IP is determined by the orientation of the optical axis of the camera, the positional relationship between the camera and the photographed object, and the camera parameter. When the camera moves and the orientation changes, the camera coordinate system moves, and the coordinate projected in the image plane IP therefore changes. Incidentally, in the explanation below, it is assumed that the parameter f is obtained beforehand.

In Fig. 11(b), there is shown a drawing in which Fig. 11(a) is projected on the XcZc plane. The blood vessel display device 1 explained in the above description is limited so that the photographing units 11, 12 cannot move in the Y-axis direction. That is, since, in the camera coordinate system shown in Fig. 11(a), it is allowed to omit the Yc-axis and to take only the Xc-axis and Zc-axis into account, the XcZc plane shown in Fig. 11(b) is used in the explanation below.

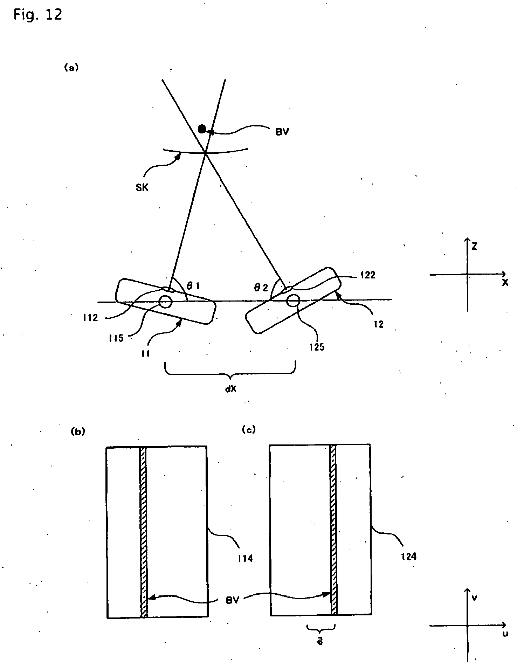

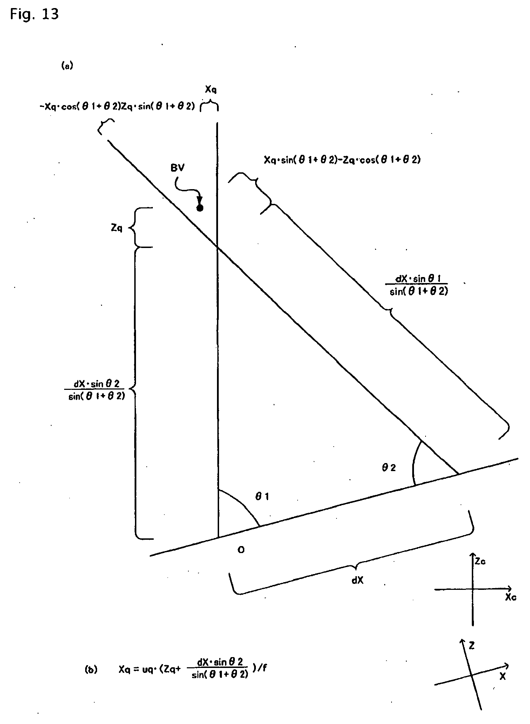

Next, an example of photographing blood vessels by means of the main photographing unit 11 and the sub-photographing unit 12 will be explained using Figs. 12(a) to 12(c). Moreover, by using Figs. 13(a) to 13(b), another explanation will be given by applying the pinhole camera model of the XcZc plane shown in Fig. 11(b) with respect to this situation of Figs. 12(a) to 12(c). Incidentally, in order to make the explanation understandable, the case in which a blood vessel parallel to the Y-axis is taken as the object is explained.

Figs. 12(a) to 12(c) are drawings showing a manner in which a skin surface is photographed by the main photographing unit 11 and the sub-photographing unit 12.

In Fig. 12(a), there are shown the main photographing unit 11 and the sub-photographing unit 12, a skin SK photographed by these photographing units 11, 12, and a blood vessel BV existing underneath the skin SK through a cross-sectional view using the XZ plane. Moreover, in Fig. 12(b), the blood vessel BV photographed by the main photographing unit 11 indicated in Fig. 12(a) is shown, and, in Fig. 12(c), the blood vessel BV photographed by the sub-photographing unit 12 indicated in Fig. 12(a) is shown. Incidentally, the difference between u-axis coordinates of the blood vessel BV respectively shown in Fig. 12(b) and Fig. 12(c) is occasionally called as a movement amount du in the below.

In Fig. 12(a), it is shown that the main photographing unit 11 is inclined by an angle θ1 with respect to the X-axis, the sub-photographing unit 12 is inclined by an angle θ2 with respect to the X-axis, and further, the main photographing unit 11 and the sub-photographing unit 12 are apart from each other in the X-axis direction by a distance dX. Furthermore, it is shown that the optical axes of the respective photographing units 11, 12 intersect to each other on the surface of the skin SK.

Here, consideration will be paid as to the camera coordinate system taking the main photographing unit 11 as the origin point by using Figs. 13(a) to 13(b). The drawing is a view in which the positional relationship in Figs. 12(a) to 12(c) is shown using the camera coordinate system having the main photographing unit at the center.

It is assumed that the blood vessel BV is the coordinate of Xq on the Xc-axis in this drawing. In addition, it is also assumed that the blood vessel BV exists at the position the distance (depth) of which is Zq from the intersection point of the Zc-axis and the skin SK along the Zc-axis. Incidentally, although the distance between the main photographing unit 11 and the sub-photographing unit 12 is not identical with the distance between the lenses 112, 122 of the respective ones, the explanation is herein continued by assuming to be identical with each other.

In Fig. 13(a), there is indicated the position of the blood vessel BV in the camera coordinate system according to the main photographing unit 11. This position is shown by the angles θ1, θ2, and the distance dX in addition to the above-mentioned Xq and Zq. Moreover, the optical axis of the sub-photographing unit 12 and the position of the blood vessel BV in the camera coordinate system on the basis of this sub-photographing unit 12 are shown by the above-mentioned parameters of Xq, Zq, the angles θ1, θ2, and the distance dX.

In the above-mentioned Figs. 11(a) to 11(b), the coordinate of the point P on the u-axis in the image plane is shown by the coordinate Zp of the Zc-axis, the coordinate Xp of the Xc-axis and the camera parameter f. That is, in Figs. 13(a) to 13(b), based on that the coordinate of the main photographing unit 11 on the u-axis in the image plane is uq, the coordinate Xq on the Xc-axis can be shown by the coordinate Zq of the Zc-axis and the camera parameter f. In Fig. 13(b), there is indicated an equation that is transformed from Xq in accordance with this expression method.

As to the angles θ1, θ2 and the distance dX among the above-mentioned parameters, it is possible to be measured by using sensors. And, as to the camera parameter, it is possible to be obtained beforehand. That is, all the equations indicated in Fig. 13(a) and the movement amount du of the blood vessel BV due to parallax can be expressed by the depth Zq of the blood vessel BV and the coordinate uq in the image plane.

Here, if an arbitrary value is assigned to Zq and the value of uq is obtained in some way, it is possible to calculate how extent the blood vessel BV has moved in between the main photographing unit 11 and the sub-photographing unit 12.

As to the coordinate uq of the blood vessel BV in the image plane, methods that the user inputs the position of the blood vessel BV actually displayed, and the position where the blood vessel BV is matched up to a specific position are thought, and a method of obtaining the position by means of image processing such as edge extraction or the like is also thought. As to the case in which a user has adjusted so that the blood vessel BV is displayed at the center of the main photographing unit 11 on the u-axis, the configuration of the second variation example of the blood vessel display device 1 for performing the above-mentioned calculation will be explained below.

This second variation example is one that is further provided with sensors and a CPU for measuring positions and orientations in the photographing units 11, 12 of the blood vessel display device 1 shown in Fig. 1, and provides information as to the depth of blood vessels to a user on the basis of information obtained from these sensors. As to the main photographing unit 11 and the sub-photographing unit 12, an explanation will be given below.

The main photographing unit 11 is provided with a position sensor (corresponding to one example of the position information obtaining means of the present invention) for obtaining the position of the main photographing unit 11 and an angle sensor (corresponding to one example of the orientation information obtaining means of the present invention) for obtaining the orientation thereof in addition to the configuration explained in the above description.

The sub-photographing unit 12 is provided with a position sensor (corresponding to one example of the position information obtaining means of the present invention) and an angle sensor (corresponding to one example of the orientation information obtaining means of the present invention) as with the main photographing unit 11. The main photographing unit 11 and the sub-photographing unit 12 are connected to each other by a cable for transmitting/receiving data. The information obtained by the sensor of the main photographing unit 11 is transmitted to the sub-photographing unit 12 by using this cable, and is used at the occasion of executing a program described later.

In the sub-photographing unit 12, there are provided a ROM that, based on the information obtained from the position sensor and the angle sensor explained in the above description, stores a program for allowing the liquid crystal display devices 114, 124 to display additional information for knowing the depth of blood vessels; a CPU (corresponding to one example of the depth information display means of the present invention) for executing this program; and a RAM that becomes the operational area of this CPU. An explanation will be given below as to this program for allowing to display the additional information.

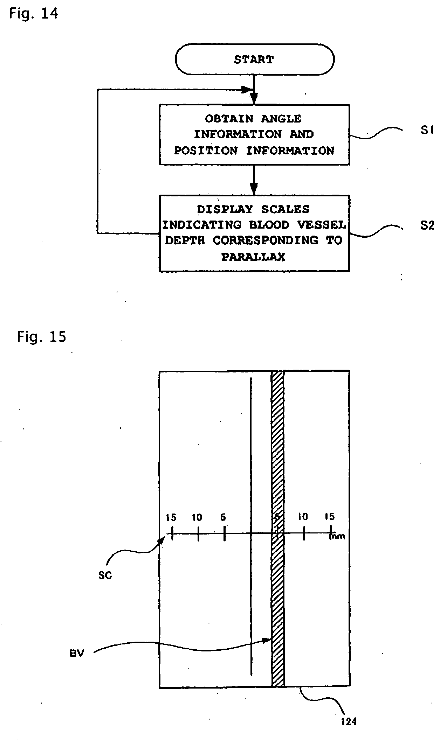

Fig. 14 is a flowchart showing the flow of the program for displaying the additional information.

Upon turning on the power source of the blood vessel display device 1, this program is executed by the above-mentioned CPU.

In step S1, information on the position and angle of each of the photographing units 11, 12 is obtained from the position sensor and angle sensor provided in each of the photographing units 11, 12.

In step S2, as the depth of a blood vessel is added in steps of 1 mm, the movement amounts corresponding to these values are calculated on the basis of the information obtained in step S1, and scales are displayed on the liquid crystal display device 124 in accordance with the calculation results. Thereafter, the flow is returned to the step S1, and the process is repeated.

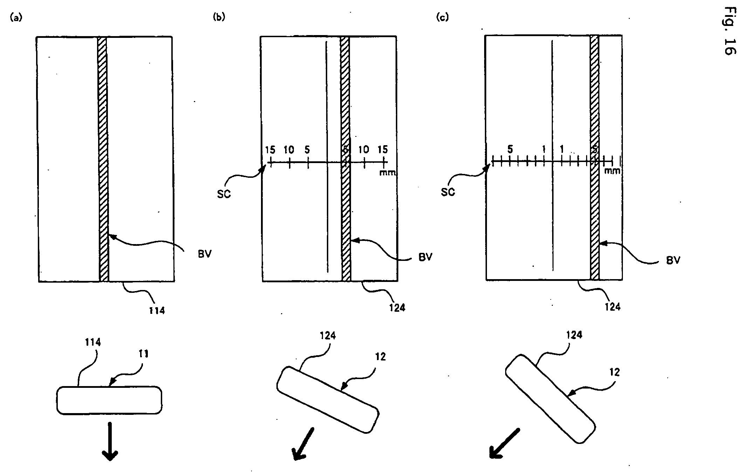

Fig. 15 is a drawing showing a manner in which scales are indicated in accordance with the flowchart shown in Fig. 14, and Figs. 16(a) to 16(c) are drawings showing a usage example of the second variation example of the blood vessel display device 1.

In Fig. 15, there is shown a manner in which a blood vessel BV is displayed on the liquid crystal display device 124 of the sub-photographing unit 12, and scales SC are displayed by being overlaid thereon. Moreover, there are shown the position and orientation of the main photographing unit 11 and also the photographed contents in Fig. 16(a), and there are shown the position and orientation of the sub-photographing unit 12 and also the photographed contents in Figs. 16(b) and (c).

When adjustment is implemented so that the blood vessel BV is displayed at the center of the liquid crystal display device 114 of the main photographing unit 11 as shown in Fig. 16(a), it is estimated from the contents of the liquid crystal display device 124 of the sub-photographing unit 12 that the blood vessel BV exists at the location of the depth 5 mm as shown in Fig. 16(b). Furthermore, when the position and orientation of the sub-photographing unit 12 are changed as shown in Fig. 16(c), renewed scales SC are displayed through the process shown in Fig. 14.

The above-mentioned second variation example is predicated on that adjustment is implemented so that the blood vessel BV is displayed at the center of the liquid crystal display device 114 of the main photographing unit 11. More specifically, it is one that the u-axis coordinate uq is set (set as 0 in the example of Figs. 16(a) to 16(c)) in the equation shown in Fig. 13(b), and the depth of the blood vessel BV is estimated after the position where the blood vessel BV is displayed is adjusted so as to agree with this coordinate.

Here, it may be allowed that an input device for inputting the position of the blood vessel BV is provided in the main photographing unit 11 and scales are displayed also on the liquid crystal display device 114 of the main photographing unit 11 by using the information inputted by this input device so that the depth of the blood vessel BV can be estimated even in the case in which the blood vessel BV is not displayed at the center of the liquid crystal display device 114 of the main photographing unit 11. That is, it may be allowed that the above-mentioned u-axis coordinate uq can be inputted. Likewise, as described above, it may also be allowed to obtain the value of the coordinate uq by using image processing.

In addition, it may also be allowed that an image displayed on the liquid crystal display device 114 of the main photographing unit 11 is displayed by being overlaid on the liquid crystal display device 124 of the sub-photographing unit 12 and the movement amount of the blood vessel BV can be easily grasped. It may also be allowed to provide a switch for interchanging the roles of the main photographing unit 11 and the sub-photographing unit 12.

In the blood vessel display device 1 explained in the above description, it is possible to provide information for estimating the depth of a blood vessel. At this occasion, since a simple calculation is employed, displaying does not require a time as with the case of using complicated image processing. As a matter of course, it may be allowed to establish correspondence between blood vessels through image processing, and to allow the depth of a blood vessel BV to be automatically calculated.

Incidentally, although a calculation method based on a pinhole camera model has been described in the explanation described above, it may be allowed that, for example, a model matched to the lens in use for a camera is employed and a program based on this model is executed.

### Reference Sings List

1 blood vessel display device
11 main photographing unit
12 sub-photographing unit
111, 121 near infrared ray LED
112, 122 lens
113, 123 visible light LED
114, 124 liquid crystal display device
115, 125 shaft
13 outer frame
131 support strut
132 guide rail
14 adjustment screw
SL slits

## Claims

1. A blood vessel display device for displaying a blood vessel existing directly underneath a skin by performing near infrared ray photographing of a surface of the skin in a state of irradiating the skin surface with near infrared rays through a near infrared ray irradiation means, the blood vessel display device comprising:
a photographing means which includes a first photographing portion having an optical system for performing near infrared ray photographing by using the optical system, and a second photographing portion having an optical system for performing near infrared ray photographing by using the optical system; at least either one of the first photographing portion and the second photographing portion is provided so that a first optical axis, which is an axis of an optical system of the first photographing portion, and a second optical axis, which is an axis of an optical system of the second photographing portion, intersect with each other on the skin surface;
an image display means which includes a first image display means configured to display a first picture, which is a near infrared ray picture photographed by the first photographing portion;
**characterised in that** it further comprises:
a first guide beam irradiation means configured to irradiate directional visible light in a direction in which the first photographing portion photographs; and
a second guide beam irradiation means configured to irradiate directional visible light in a direction in which the second photographing portion photographs;
wherein
the image display means further includes a second image display means configured to display a second picture, which is a near infrared ray picture photographed by the second photographing portion,
the first photographing portion and the first image display means are integrally constituted on both sides,
the second photographing portion and the second image display means are integrally constituted on both sides,
at least either one of a relative relationship between the position of the first optical axis and the position of the second optical axis and a relative relationship between the direction of the first optical axis and the direction of the second optical axis is possible to be changed, and
by allowing visible light irradiated from the first guide beam irradiation means and visible light irradiated from the second guide beam irradiation means to intersect with each other on a skin surface, the first optical axis and the second optical axis are enabled to intersect with each other on the skin surface.

2. The blood vessel display device according to claim 1,
wherein the first photographing portion and the second photographing portion are arranged so that the first optical axis and the second optical axis are included in optical path planes, which are an identical plane, and
at least either one of the first photographing portion and the second photographing portion is provided so that at least either one of a relative relationship between the position of the first optical axis and the position of the second optical axis and a relative relationship between the direction of the first optical axis and the direction of the second optical axis is possible to be changed while a state in which the first optical axis and the second optical axis are included in the optical path plane is maintained.

3. The blood vessel display device according to claim 2,
further comprising an adjustment mechanism for changing positions and directions of both the first optical axis and the second optical axis while maintaining the relative relationship between the position of the first optical axis and the position of the second optical axis and also the relative relationship between the direction of the first optical axis and the direction of the second optical axis.

4. The blood vessel display device according to claim 2 or 3 further comprising:
a position information obtaining means configured to obtain information of a position of the first optical axis and a position of the second optical axis;
a direction information obtaining means configured to obtain information of a direction of the first optical axis and a direction of the second optical axis; and
a depth information display means configured to display in a state in which the first optical axis and the second optical axis intersect with each other at an identical place on the skin surface, information for estimating the depth of a blood vessel existing underneath the identical place on either one of the first image display means and the second image display means by using information obtained through the position information obtaining means and the direction information obtaining means.

5. The blood vessel display device according to claim 4, wherein the depth information display means is configured to display information for estimating the depth of a blood vessel, which exists at the identical place and also exists on the optical axis of either one of the first optical axis and the second optical axis, on either one of the first image display means and the second image display means by using the information obtained through the position information obtaining means and the direction information obtaining means.

6. The blood vessel display device according to claim 4 further comprising:
an input means configured to input as to a near infrared ray picture displayed on either one of the first image display means and the second image display means, positional information of a blood vessel existing underneath the identical place in the picture, wherein
the depth information display means is configured to display information obtained through the position information obtaining means and the direction information obtaining means, and information for estimating the depth of the blood vessel by using the blood vessel positional information inputted by the input means on either one of the first image display means and the second image display means.

## Patentansprüche

1. Blutgefäß-Anzeigevorrichtung zum Anzeigen eines Blutgefäßes direkt unterhalb der Haut durch Ausführen einer Nahinfrarotstrahlungs-Fotografie einer Hautoberfläche in einem Zustand, in welchem die Hautoberfläche mit Strahlen im nahen Infrarotbereich mit Hilfe einer Nahinfrarotstrahlen-Bestrahlungseinrichtung bestrahlt wird, wobei die Blutgefäß-Anzeigevorrichtung aufweist:
eine Fotografiereinrichtung, die einen ersten Fotografierabschnitt mit einer Optik zum Ausführen einer Nahinfrarotstrahlungs-Fotografie unter Verwendung der Optik und einen zweiten Fotografierabschnitt mit einer Optik zum Ausführen einer Nahinfrarotstrahlung-Fotografie unter Verwendung der Optik aufweist; wobei mindestens einer von dem ersten Fotografierabschnitt und dem zweiten Fotografierabschnitt derart vorgesehen ist, dass eine erste optische Achse, nämlich eine Achse einer Optik des ersten Fotografierabschnitts, und eine zweite optische Achse, nämlich eine Achse einer Optik des zweiten Fotografierabschnitts, einander auf der Hautoberfläche schneiden;
eine Bildanzeigeeinrichtung, die eine erste Bildanzeigeeinrichtung, konfiguriert zum Anzeigen eines ersten Bilds, bei dem es sich um das von dem ersten Fotografierabschnitt fotografierte Nahinfrarotstrahlenbild handelt, enthält;
**dadurch gekennzeichnet, dass** sie weiterhin umfasst:
eine erste Führungsstrahleinrichtung, konfiguriert zum Strahlen von sichtbarem Richtungslicht in eine Richtung, in der der erste Fotografierabschnitt fotografiert; und
eine zweite Führungsstrahlreinrichtung, konfiguriert zum Strahlen von sichtbarem Richtungslicht in eine Richtung, in der der zweite Fotografierabschnitt fotografiert; wobei
die Bildanzeigeeinrichtung weiterhin eine zweite Bildanzeigeeinrichtung enthält, konfiguriert zum Anzeigen eines zweiten Bilds, bei dem es sich um ein von dem zweiten Fotografierabschnitt fotografiertes Nahinfrarotstrahlenbild handelt,
der erste Fotografierabschnitt und die erste Bildanzeigeeinrichtung integral beidseitig ausgebildet sind,
der zweite Fotografierabschnitt und die zweite Bildanzeigeeinrichtung integral beidseitig ausgebildet sind,
mindestens eine von einer Relativlagebeziehung zwischen der Position der ersten optischen Achse und der Position der zweiten optischen Achse und einer Relativlagebeziehung zwischen der Richtung der ersten optischen Achse und der Richtung der zweiten optischen Achse änderbar ist, und
durch Ermöglichen, dass von der ersten Führungsstrahleinrichtung abgestrahltes sichtbares Licht und von der zweiten Führungsstrahleinrichtung abgestrahltes sichtbares Licht einander auf einer Hautoberfläche schneiden, die erste optische Achse und die zweite optische Achse einander auf der Hautoberfläche schneiden können.

2. Blutgefäß-Anzeigevorrichtung nach Anspruch 1, bei der der erste Fotografierabschnitt und der zweite Fotografierabschnitt derart angeordnet sind, dass die erste optische Achse und die zweite optische Achse in optischen Wegebenen enthalten sind, die eine identische Ebene bilden, und
mindestens einer von dem ersten Fotografierabschnitt und dem zweiten Fotografierabschnitt derart vorgesehen ist, dass mindestens eine von einer Relativlagebeziehung zwischen der Position der ersten optischen Achse und der zweiten optischen Achse, und einer Relativlagebeziehung zwischen der Richtung der ersten optischen Achse und der Richtung der zweiten optischen Achse änderbar ist, während ein Zustand, in welchem die erste optische Achse und die zweite optische Achse in der optischen Wegebene enthalten sind, aufrecht erhalten bleibt.

3. Blutgefäß-Anzeigevorrichtung nach Anspruch 2, weiterhin umfassend einen Einstellmechanismus zum Ändern von Positionen und Richtungen sowohl der ersten als auch der zweiten optischen Achse unter gleichzeitiger Aufrechterhaltung der Relativlagebeziehung zwischen der Position der ersten optischen Achse und der Position der zweiten optischen Achse, und außerdem der Relativlagebeziehung zwischen der Richtung der ersten optischen Achse und der Richtung der zweiten optischen Achse.

4. Blutgefäß-Anzeigevorrichtung nach Anspruch 2 oder 3, weiterhin umfassend:
eine Positionsinformations-Gewinnungseinrichtung, konfiguriert zum Gewinnen von Informationen über eine Position der ersten optischen Achse und einer Position der zweiten optischen Achse;
eine Richtungsinformations-Gewinnungseinrichtung, konfiguriert zum Gewinnen von Information über eine Richtung der ersten optischen Achse und einer Richtung der zweiten optischen Achse; und
eine Tiefeninformations-Anzeigeeinrichtung, konfiguriert, um in einen Zustand, in welchem die erste optische Achse und die zweite optische Achse einander an einer identischen Stelle auf der Hautoberfläche schneiden, Information zum Abschätzen der Tiefe eines Blutgefäßes anzuzeigen, welches sich unterhalb der identischen Stelle auf einer von der ersten Bildanzeigeeinrichtung und der zweiten Bildanzeigeeinrichtung befindet, indem die über die Positionsinformations-Gewinnungseinrichtung und die Richtungsinformations-Gewinnungseinrichtung gewonnene Information genutzt wird.

5. Blutgefäß-Anzeigevorrichtung nach Anspruch 4, bei der die Tiefeninformations-Anzeigeeinrichtung konfiguriert ist zum Anzeigen von Information zum Abschätzen der Tiefe eines Blutgefäßes, welches sich an der identischen Stelle befindet, und das sich außerdem auf der optischen Achse entweder der ersten optischen Achse oder zweiten optischen Achse befindet, und zwar auf entweder der ersten Bildanzeigeeinrichtung oder der zweiten Bildanzeigeeinrichtung, indem die Information genutzt wird, die von der Positionsinformations-Gewinnungseinrichtung und der Richtungsinformations-Gewinnungseinrichtung gewonnen wurde.

6. Blutgefäß-Anzeigevorrichtung nach Anspruch 4, weiterhin umfassend:
eine Eingabeeinrichtung, konfiguriert, um zu einem auf entweder der ersten Bildanzeige-Einrichtung oder der zweiten Bildanzeige-Einrichtung angezeigten Nahinfrarotstrahlenbild Positionsinformation eines Blutgefäßes einzugeben, welches sich unterhalb der identischen Stelle in dem Bild befindet, wobei
die Tiefeninformations-Anzeigeeinrichtung konfiguriert ist zum Anzeigen von Information, die über die Positionsinformations-Gewinnungseinrichtung und die Richtungsinformations-Gewinnungseinrichtung gewonnen wurde, außerdem Information zum Abschätzen der Tiefe des Blutgefäßes unter Verwendung der Blutgefäß-Positionsinformation, die von der Eingabeeinrichtung auf entweder der ersten Bildanzeigeeinrichtung oder der zweiten Bildanzeigeeinrichtung eingegeben wurde.

## Revendications

1. Dispositif d'affichage de vaisseau sanguin destiné à afficher un vaisseau sanguin existant directement sous une peau en réalisant une photographie à rayonnement infrarouge proche d'une surface de la peau dans un état d'irradiation de la surface de peau avec des rayons infrarouges proches par le biais d'un moyen d'irradiation à rayonnement infrarouge proche, le dispositif d'affichage de vaisseau sanguin comprenant :
un moyen de photographie qui inclut une première partie de photographie présentant un système optique destiné à réaliser une photographie à rayonnement infrarouge proche en utilisant le système optique, et une seconde partie de photographie présentant un système optique destiné à réaliser une photographie à rayonnement infrarouge proche en utilisant le système optique, l'une ou l'autre au moins parmi la première partie de photographie et la seconde partie de photographie étant fournie de manière à ce qu'un premier axe, qui est un axe d'un système optique de la première partie de photographie, et un second axe optique, qui est un axe d'un système optique de la seconde partie de photographie, forment entre eux une intersection sur la surface de peau,
un moyen d'affichage d'image qui inclut un premier moyen d'affichage d'image configuré pour afficher une première image, laquelle est une image de rayonnement infrarouge proche photographiée par la première partie de photographie ;
**caractérisé en ce qu'**il comprend en outre :
un premier moyen d'irradiation de faisceau de guidage configuré pour irradier une lumière visible directionnelle dans une direction dans laquelle la première partie de photographie photographie, et
un second moyen d'irradiation de faisceau de guidage configuré pour irradier une lumière visible directionnelle dans une direction dans laquelle la seconde partie de photographie photographie ;
dans lequel
le moyen d'affichage d'image inclut en outre un second moyen d'affichage d'image configuré pour afficher une seconde image, laquelle est une image de rayonnement infrarouge proche photographiée par la seconde partie de photographie ;
la première partie de photographie et le premier moyen d'affichage d'image sont constitués d'un seul tenant sur les deux côtés ;
la seconde partie de photographie et le second moyen d'affichage d'image sont constitués d'un seul tenant sur les deux côtés ;
il est possible de modifier au moins l'une ou l'autre relation parmi une relation relative entre la position du premier axe optique et la position du second axe optique, et une relation relative entre la direction du premier axe optique et la direction du second axe optique, et
en permettant l'intersection entre une lumière visible irradiée à partir du premier moyen d'irradiation de faisceau de guidage et une lumière visible irradiée à partir du second moyen d'irradiation de faisceau de guidage sur une surface de peau, le premier axe optique et le second axe optique sont en mesure de former une intersection entre eux sur la surface de peau.

2. Dispositif d'affichage de vaisseau sanguin selon la revendication 1,
dans lequel la première partie de photographie et la seconde partie de photographie sont agencées de telle sorte que le premier axe optique et le second axe optique sont inclus dans des plans de trajet optique, lesquels ont un plan identique, et
au moins l'une ou l'autre parmi la première partie de photographie et la seconde partie de photographie est fournie de telle sorte qu'il est possible de modifier au moins l'une ou l'autre relation parmi une relation relative entre la position du premier axe optique et la position du second axe optique, et une relation relative entre la direction du premier axe optique et la direction du second axe optique tandis qu'est maintenu un état dans lequel le premier axe optique et le second axe optique sont inclus dans le plan de trajet optique.

3. Dispositif d'affichage de vaisseau sanguin selon la revendication 2,
comprenant en outre un mécanisme d'ajustement destiné à modifier des positions et directions à la fois du premier axe optique et du second axe optique, tout en maintenant la relation relative entre la position du premier axe optique et la position du second axe optique, ainsi que la relation relative entre la direction du premier axe optique et la direction du second axe optique.

4. Dispositif d'affichage de vaisseau sanguin selon la revendication 2 ou 3, comprenant en outre :
un moyen d'obtention d'informations de position configuré pour obtenir des informations d'une position du premier axe optique et d'une position du second axe optique ;
un moyen d'obtention d'informations de direction configuré pour obtenir des informations d'une direction du premier axe optique et d'une direction du second axe optique, et
un moyen d'affichage d'informations de profondeur configuré pour afficher, dans un état où le premier axe optique et le second axe optique forment une intersection entre eux en un endroit identique sur la surface de peau, des informations destinées à estimer la profondeur d'un vaisseau sanguin existant sous un endroit identique sur l'un ou l'autre du premier moyen d'affichage d'image et du second moyen d'affichage d'image en utilisant des informations obtenues par le biais du moyen d'obtention d'informations de position et du moyen d'obtention d'informations de direction.

5. Dispositif d'affichage de vaisseau sanguin selon la revendication 4, dans lequel le moyen d'affichage d'informations de profondeur est configuré pour afficher des informations destinées à estimer la profondeur d'un vaisseau sanguin, lequel existe à l'endroit identique et existe également sur l'axe optique de l'un ou l'autre parmi le premier axe optique et le second axe optique, sur l'un ou l'autre du premier moyen d'affichage d'image et du second moyen d'affichage d'image, en utilisant des informations obtenues par le biais du moyen d'obtention d'informations de position et du moyen d'obtention d'informations de direction.

6. Dispositif d'affichage de vaisseau sanguin selon la revendication 4, comprenant en outre :
un moyen d'entrée configuré pour entrer, concernant une image de rayonnement infrarouge proche affichée sur l'un ou l'autre du premier moyen d'affichage d'image et du second moyen d'affichage d'image, des informations de position d'un vaisseau sanguin existant sous l'endroit identique dans l'image, dans lequel
le moyen d'affichage d'informations de profondeur est configuré pour afficher des informations obtenues par le biais du moyen d'obtention d'informations de position et du moyen d'obtention d'informations de direction, et des informations destinées à estimer la profondeur du vaisseau sanguin en utilisant les informations de position de vaisseau sanguin entrées par le moyen d'entrée sur l'un ou l'autre du premier moyen d'affichage d'image et du second moyen d'affichage d'image.
